# EUROPEAN PATENT APPLICATION

(11) **EP 4 328 307 A1**
(43) Date of publication of application: **28.02.2024**
(21) Application number: 22790655.9
(22) Date of filing: 22.04.2022
(51) Int. Cl.: C12N 15/10, C12N 5/071, C12N 5/074

(54) **EXTRACELLULAR VESICLES OF UMBILICAL CORD MESENCHYMAL CELLS FOR TREATING OSTEOARTICULAR AND AUTOIMMUNE DISEASES**

(30) Priority: 22.04.2021 US 202163178229 P; 12.04.2022 US 202263330092 P
(71) Applicant: Consorcio Regenero, S.A., Santiago, 7550101 (CL)
(72) Inventor: KHOURY, Maroun, Santiago, 7550101 (CL); ALCAYAGA, Francisca, Santiago, 7550101 (CL); FIGUEROA, Aliosha, Santiago, 7550101 (CL)
(74) Representative: Elzaburu S.L.P.
(86) International application number: PCT/CL2022/050039
(87) International publication number: WO 2022/221969

(57) **Abstract**

Composition enriched in extracellular vesicles (EVs) of umbilical cord mesenchymal cells, with specific markers and miRNAs, where this composition is useful in the treatment of osteoarticular and autoimmune diseases.

Specifically, an enriched composition in extracellular vesicles (EVs) of umbilical cord mesenchymal cells is protected, which consists of a set of EVs containing the miRNAs: hsa-miR-6126, hsa-miR-149-3p and hsa-miR-6780b-5p, among the 10 most abundant miRNAs, and additionally contain at least 5 of the following miRNAs: hsa-miR-574-5p, hsa-miR-6131, hsa-miR-6741-5p, hsa-miR-21-5p, hsa-miR-1290, hsa-miR-4530, hsa-miR-2861, hsa-miR-6088, hsa-miR-1307-5p, and/or hsa-miR-6782-5p, and have surface markers CD63, CD81, and CD9; and a medium or vehicle free of animal components and proteins, with an osmolality of between 250 and 310 mOsmol/L and a pH between 6.0 and 8.0. Method of obtaining the composition and its use in the treatment of osteoarticular and autoimmune diseases.

## Description

### Technical field.

The invention relates to the general field of biotechnology, and specifically relates to the field of regenerative medicine and the manufacture of therapy products based on human mesenchymal cells, specifically extracellular vesicles, for the treatment of osteoarticular and autoimmune diseases.

### State of the Art

Currently, cell therapy forms a bridge between research and clinical application and constitutes an advance with respect to tissue engineering in order to replace, regenerate and/or modify human cells, tissues or organs in order to recover functionality. This translation is accompanied and encouraged by a growing number of clinical trials using stem cells, mainly mesenchymal stem cells (MSCs). Although the field of cell therapy has advanced rapidly in various medical applications, in its development there are several technical problems that require better solutions, which allow on the one hand to lower the costs of these technologies, and also make it more efficient and safer, so as to be able to help more people and in new applications.

A recent approach in this regard, which is safer and less costly, is to use vesicles naturally secreted by cells during cell culture, as they have been shown to have great potential for cell-free therapies. Researchers have studied the function of these vesicles in various areas, including that they play an important role in intercellular communication, that they have an effect on the formation and/or modulation of diseases through their content, which corresponds essentially to proteins and RNA. It has been especially found that these vesicles contain a high number and variety, between 2000 to 3000 types of microRNA or miRNA. These molecules are non-coding RNA strands, which play an important role in regulating gene expression. A subset of extracellular vesicles are exosomes (30 to 200 nm), which have already been used as vehicles for the delivery of different drugs.

Currently, the development of therapies based on extracellular vesicles, including exosomes or small extracellular vesicles (sEVs), has advances in preclinical studies and great potential for clinical application, such as those proposed in this invention.

In the state of the art there are different diagnostic applications and some treatment with isolated exosomes, for example, the publication WO2019038660A1 (Seattle Childrens Hospital DBA Seattle Childrens Res Inst, 2019-02-28), protects a method of diagnosing Kawasaki disease by examining the exosomes present in the patient's biological fluids.

There are some patent applications in which exosomes of mesenchymal cells are used for the treatment of some diseases, such as glaucoma, US20190224242A1 (Tomarev et al,2019-07-25), but the composition used and the properties observed in that document are far from the composition of the invention, for example in the application exosomes have the markers CD11c+ and CD63+, while in invention the markers CD63, CD81 and CD9 are required, to name just one difference.

Inventors have not found in previous art any document anticipating the invention. Thus, the invention aims at a composition of exosomes obtained from umbilical cord mesenchymal cells (UC-MSC) which contain miRNAs and/or specific surface markers, for the treatment of osteoarticular and autoimmune diseases.

### Description of the figures.

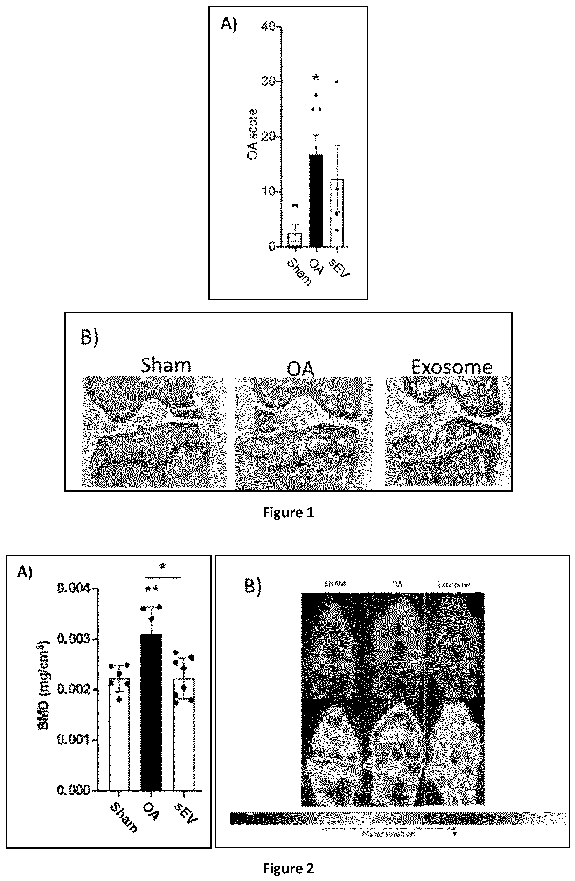

**Figure 1****.** Treatment with the composition of the invention allows for reduction of the severity of the lesion, in a murine model of osteoarthritis, Study 1. A) OA histology score (or OA score) shows a healthy joint (Sham, or placebo), a joint with osteoarthritis, and a joint with osteoarthritis treated with composition of the invention (exosome). B) Representative image showing histological sections of the "sham" control knee (left), the knee with OA induction (center) and the knee with treatment with the composition of the invention, exosomes (right). A higher proportion of subchondral bone was observed in the knee treated with exosomes p < 0.05 in comparison to the OA group.
**Figure 2****.** Treatment with the composition of the invention shows positive results in bone mineral density in murine model of osteoarthritis, Study 2. A) Analysis of the bone mineral density of the medial tibial condyle of the knee, which corresponds to a histomorphometric analysis of the 3D images. B) Representative image showing images of the medial tibial condyle observed by µCT of the knee of the "sham" control (left), of the knee with OA induction (center) and of the knee with exosome treatment (right), along with a color scale representing mineralization, with the far left being the valuation with the lowest mineralization and the far right being the valuation with the highest mineralization. It is observed that treatment with exosomes prevents further bone mineralization since the colors observed regarding the degree of mineralization are similar to the colors obtained in the "sham" control, that is, colors that are on the left of the scale described above.

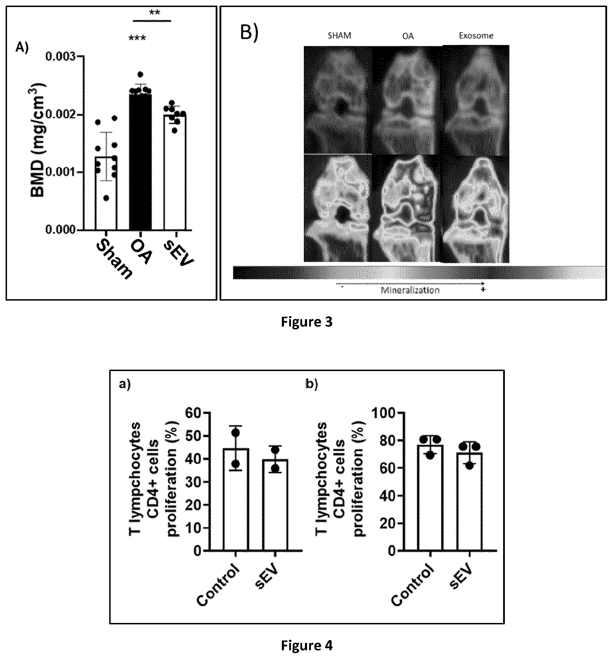

**Figure 3****.** Treatment with the composition of the invention shows positive results in bone mineral density in murine model of osteoarthritis, Study 3. A) Analysis of the bone mineral density of the medial tibial condyle of the knee, which corresponds to a histomorphometric analysis of the 3D images. B) Representative image showing images of the medial tibial condyle observed by µCT of the knee of the "sham" control (left), of the knee with OA induction (center) and of the knee with exosome treatment (right), along with a color scale representing mineralization, with the far left being the valuation with the lowest mineralization and the far right being the valuation with the highest mineralization. It is observed that treatment with exosomes prevents further bone mineralization since the colors observed regarding the degree of mineralization are similar to the colors obtained in the "sham" control, that is, colors that are on the left of the scale described above.
**Figure 4****.** Treatment with the composition of the invention decreases the proliferation of CD4+ Th cells. A) peripheral blood mononuclear cells, activated with PHA (20µg/mL), treated with PBS control and with the composition of the invention, the proliferation of CD4+ Th cells is shown on the third day of culture. B) proliferation on the fifth day. The composition of the invention results in less proliferation than the control at both times.

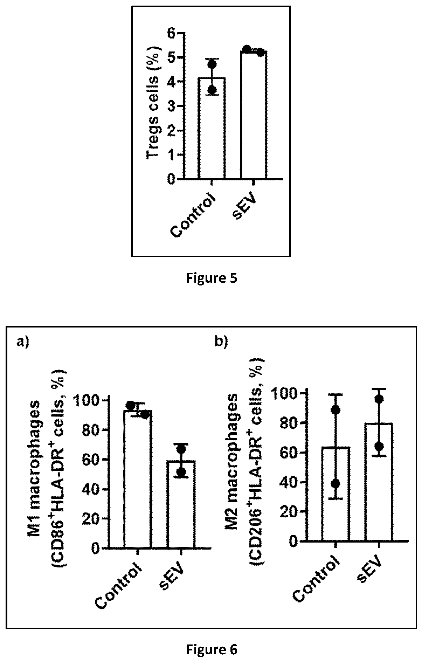

**Figure 5****.** Treatment with the composition of the invention increases the proliferation of Treg cells; characterized by possessing the markers CD4, FOXP3 and CD25. Peripheral blood mononuclear cells, activated with PHA (20µg/mL), treated with PBS control and with the composition of the invention, the proliferation of Tregs cells is shown on the third day of culture. The composition of the invention allows for greater proliferation than the control.
**Figure 6****.** Treatment with the composition of the invention positively polarizes the differentiation of macrophages. Monocytes were isolated from peripheral blood mononuclear cells, which were cultured and differentiated into macrophages by the factors M-CSF and GM-CSF, at 6 days they were treated with PBS control and with the composition of the invention, A) proliferation of M1 or pro-inflammatory macrophages 24 hours after the stimulus, is decreased by the composition of the sEV invention, B) proliferation of M2 macrophages or anti-inflammatories 24 hours after stimulation is increased by the composition of the sEV invention.

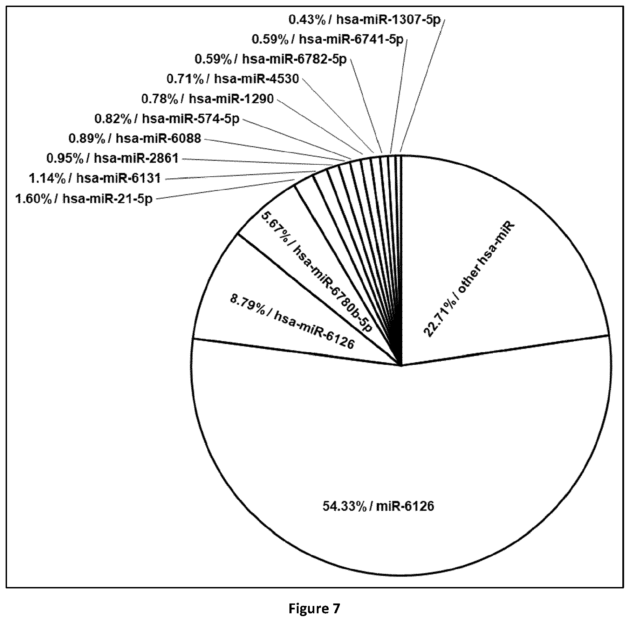

**Figure 7****.** Graph showing the relative concentrations of hsa-miRNAs in the extracellular vesicles present in the composition of the invention, for reasons.

### Description of the invention

The invention refers to a composition enriched in extracellular vesicles (EVs) of umbilical cord mesenchymal cells, with specific markers and miRNAs, where this composition is useful in the treatment of osteoarticular and autoimmune diseases, especially when injected into the sites of the lesion to be treated.

Specifically, the invention targets an enriched composition in extracellular vesicles (EVs) of umbilical cord mesenchymal cells, which consists of a set of EVs containing the miRNAs: hsa-miR-6126, hsa-miR-149-3p and hsa-miR-6780b-5p, among the 10 most abundant miRNAs, and additionally contain at least 5 of the following miRNAs: hsa-miR-574-5p, hsa-miR-6131, hsa-miR-6741-5p, hsa-miR-21-5p, hsa-miR-1290, hsa-miR-4530, hsa-miR-2861, hsa-miR-6088, hsa-miR-1307-5p and/or hsa-miR-6782-5p, and have the surface markers CD63, CD81, and CD9;
- and a medium or vehicle free of animal components and proteins, with an osmolality of between 250 and 310 mOsmol/L and a pH between 6.0 and 8.0.

In addition, vesicles or exosomes contain the following miRNAs: hsa-miR-8078; hsa-miR-7150; hsa-miR-6750-5p; hsa-miR-6727-5p; hsa-miR-5585-3p; hsa-miR-4459; hsa-miR-4449; hsa-miR-3687; hsa-miR-3197; hsa-miR-1915-3p; hsa-miR-1285-5p; hsa-miR-1275; hsa-miR-1273h-5p; hsa-miR-1273d; hsa-miR-1237-5p; hsa-miR-874-3p; hsa-miR-671-5p; hsa-miR-424-5p; hsa-miR-376c-3p; hsa-miR-339-3p; hsa-miR-222-3p; hsa-miR-221-3p; hsa-miR-199a-3p; hsa-miR-193a-3p; hsa-miR-181a-5p; hsa-miR-146a-5p; hsa-miR-145-5p; hsa-miR-143-3p; hsa-miR-130a-3p; hsa-miR-125b-5p; hsa-miR-125a-5p; hsa-miR-100-5p; hsa-miR-99a-5p; hsa-miR-92a-3p; hsa-miR-31-5p; hsa-miR-29b-3p; hsa-miR-29a-3p; hsa-miR-27b-3p; hsa-miR-27a-3p; hsa-miR-26a-5p; hsa-miR-24-3p; hsa-miR-23b-3p; hsa-miR-23a-3p; hsa-miR-22-3p; hsa-miR-19b-3p; hsa-miR-16-5p and/or hsa-let-7i-5p, among the 100 most abundant miRNAs.

As mentioned above, it is known that the EV contains an enormous variety of miRNAs, in very different proportions, for the expert in the technique it is evident that the subgroup of the 100 most abundant miRNAs is small, since concentrations of around 2000 to 3000 miRNAs are usually sequenced and calculated. The inventors have obtained cultures of specific umbilical cord mesenchymal cells (UC-MSCs) from different donors, from which they have obtained the compositions of the invention that replicate the presence of specific markers, and the relative concentrations of miRNA indicated. These invention compounds have proven to be useful in the treatment of osteoarticular lesions and to have a positive effect on models correlated with the treatment of autoimmune diseases, as will be demonstrated in the examples.

In a second embodiment, the vesicles of the composition of the invention contain at least 8 of the following miRNAs: hsa-miR-574-5p, hsa-miR-6131, hsa-miR-6741-5p, hsa-miR-21-5p, hsa-miR-1290, hsa-miR-4530, hsa- miR-2861, hsa-miR-6088, hsa-miR-1307-5p and/or hsa-miR-6782-5p, among the 100 most abundant miRNAs.

In addition, vesicles may contain the surface markers N-cadherin and/or CD90 and/or CD44.

In the composition of the invention, vesicles are in a concentration of between 5×10⁷ to 1×10¹³ total particles per mL, and have a size between 30 and 300 nm.

In a second aspect, the invention refers to the method of obtaining the composition of the invention, in which the EVs are isolated from the supernatant of the culture of umbilical cord mesenchymal cells expanded in vitro, where these cells express at least 2 of the surface markers N-cadherin, CD44, RANKL, CD105, CD56 and/or CD90 and are resuspended in a medium or vehicle free of animal components and proteins, with an osmolality between 250 and 310 mOsmol/L and a pH between 6.0 and 8.0. In the state of the art, there are many means that meet these conditions, both commercial means and means that can be prepared in the laboratory. For example, we can name phosphate buffer or PBS, lactated Ringer, Hypotemerosol ^{®}, Plasmalyte ^{®}, to name a few. As indicated, the essential thing is the osmolality and pH, already indicated.

In addition, the EVs are isolated from the supernatant by filtering and/or centrifugation, washed at least 2 times with a medium or vehicle free of animal components and proteins, with an osmolality of between 250 and 310 mOsmol/L and a pH between 6.0 and 8.0, and finally resuspended in a medium or vehicle free of animal components and proteins, with an osmolality between 250 and 310 mOsmol/L and a pH between 6.0 and 8.0. For the expert in the technique, it will be evident that there may be different protocols to isolate the extracellular vesicles of the supernatant from the culture of mesenchymal cells. The process of the invention does not depend on the protocol or system used for this separation, as long as it is efficient in separating vesicles of a size between 30 and 300 nm, so this separation can be carried out by any technique or instrument available at the time of making the invention.

Umbilical cord mesenchymal cells from which EVs are obtained express at least 3 of the surface markers N-cadherin, CD44, RANKL, CD105, CD56 and/or CD90.

The composition of the invention has specific activities, which make it useful for preparing a drug for the treatment of osteoarticular and autoimmune diseases. For example, the composition has been shown to promote cartilage regeneration; as well as the increase in bone mineral density in clinical cases of osteoarthritis in vivo.

Moreover, the composition is useful for decreasing the proliferation of CD4+ T cells, and at the same time increasing the proliferation of Tregs cells. The composition allows for an increase in M2 macrophages, or CD206+ HLA-DR+ anti-inflammatory macrophages, and/or a decrease in M1 macrophages, or pro-inflammatory macrophages or CD86+HLA-DR+.

All these faculties, in addition to the demonstration included in the examples, make it evident that the composition is useful for the treatment of osteoarthritis, arthritis, osteoarthritis, for example. And additionally, it is useful for the treatment of rheumatoid arthritis, systemic lupus erythematosus, inflammatory bowel disease, multiple sclerosis, type 1 diabetes mellitus, Guillain-Barre syndrome, chronic inflammatory demyelinating polyneuropathy, psoriasis, Graves' disease, Hashimoto's thyroiditis, myasthenia gravis, vasculitis, Sjögren's syndrome, pernicious anemia disease, celiac disease, or graft-versus-host disease.

### Examples

### Example 1: Obtaining the composition of the invention.

Umbilical cord mesenchymal cells were obtained and found to have the markers N-cadherin, CD44, RANKL, CD105, CD56 and/or CD90. 3 batches of cells were obtained, in Table 1, below, the markers of mesenchymal cells, and the selection condition of the invention, for each of them are shown.

**Table 1. Umbilical cord mesenchymal cells used.**

| **R Marking** | **Referential Expression Level (% of cells +)** | **Lot I (%of cells+)** | **Lot II (%of cells +)** | **Lot III (% of cells +)** |
|---|---|---|---|---|
| **CD73** | > 95 | 100.00 | 99.90 | 100.00 |
| **CD90** | > 95 | 100.00 | 100.00 | 100.00 |
| **CD105** | > 95 | 99.90 | 99.90 | 100.00 |
| **CD45** | | | 0.23 | |
| **CD34** | | | 0.69 | |
| **CD11b** | < 2 | 0.01 | | 0.03 |
| **CD19** | | | 0.06 | |
| **HLA-DR** | | | 0.04 | |
| | | | 0.09 | |
| **CD44** | + | 100.00 | 100.00 | 99.70 |
| **CD254 (RANKL)** | + | 93.80 | 19.70 | 2.54 |
| **CD56** | + | 0.73 | 50.0 | 34.90 |
| **CD325 (N-Cadherin)** | + | 100.00 | 92.8 | 5.05 |

The cells thus validated were cultured under the usual conditions of any mesenchymal cell culture, up to confluence in a total volume of 650 mL, with a commercial medium (high glucose DMEM; supplemented with 5% hPL, 1% L-Glut, 1% Pen/Strep). Subsequently, the cells were washed 2 times with PBS (phosphate buffer, pH7.2; 290mOsm/L) and then 650mL of induction medium (high glucose DMEM; supplemented with only 1% L-Glut) was added.

After 48 hours of incubation, all the supernatant medium was collected from the cells, and 650mL of induction medium was added again to the culture for a second incubation of 48 hours.

The supernatant collected after each incubation 1 or 2 was centrifuged 600 g 4°C for 10 minutes to remove impurities.

The supernatants from the previous centrifugation were processed in an ultracentrifuge at 100,000 g for 1 hour and 10 minutes at 4°C, this step was repeated until the total volume of the supernatant was processed. The supernatant was removed and the pellets were released using a vortex. Subsequently, the pellets were washed twice in order to remove possible contaminants. To do this, the pellets were resuspended in filtered PBS reaching a final volume of 10 ml, and centrifuged at 100,000g at 4°C over night. Subsequently, the last wash was carried out, for which the supernatants were removed and the pellets were resuspended in a final volume of 10ml Ringer Lactate (pH 6.5, 273mOsm/L) and processed for 1 hour and 10 minutes at 4°C in an ultracentrifuge at 100,000 g. Finally, the supernatant was removed, the pellets passed through a vortex and were aliquoted for storage.

The aliquot particles were then quantified. Particle quantification was done using a pellet dilution in filtered PBS (1:100), using NanoSight NS300.

From the 3 initial cultures, 4 batches of aliquot particles or composition of the invention were obtained. Table 2 below shows the characterization of these particles in terms of the concentration obtained and the presence of validation markers.

**Table 2. Characterization of compositions according to the invention.**

| **Lot** | **Size Fashion (nm)** | **Concentration (particles/mL)** | **% CD63** | **% CD81** | **% CD9** |
|---|---|---|---|---|---|
| A | 136.2 | 3.04×10¹¹ | 99.7 | 93.9 | 86.4 |
| B | 140.2 | 1.42×10¹¹ | 95.6 | 85.9 | 68.5 |
| C | 119.3 | 1.06×10¹¹ | 97.3 | 69.3 | 16.5 |
| D | 110.6 | 7.54×10¹⁰ | 97.1 | 31.4 | 86.6 |

The 4 batches obtained were additionally analyzed with respect to identity and relative concentration of the miRNAs they contained, approximately 2500 miRNAs were sequenced in each of them. Table 3 shows the concentrations of the most relevant miRNAs in the invention. Figure 7 graphs the concentrations of Lot A.

**Table 3. miRNAs present in EVs of the composition of the invention.**

| **Mima** | **Lot A** | **Lot B** | **Lot C** | **Lot D** |
|---|---|---|---|---|
| ***hsa-miR-6126*** | 54.33% | 47.05% | 51.00% | 62.31% |
| ***hsa-miR-149-3p*** | 8.79% | 8.09% | 7.66% | 9.59% |
| ***hsa-miR-6780b-5p*** | 5.67% | 5.53% | 6.66% | 4.32% |
| ***hsa-miR-21-5p*** | 1.60% | 1.55% | 2.10% | 1.57% |
| ***hsa-miR-6131*** | 1.14% | 0.88% | 1.14% | 1.99% |
| ***hsa-miR-2861*** | 0.95% | 1.39% | 0.92% | 0.70% |
| ***hsa-miR-6088*** | 0.89% | 1.15% | 0.55% | 0.48% |
| ***hsa-miR-574-5p*** | 0.82% | 1.36% | 0.99% | 0.41% |
| ***hsa-miR-1290*** | 0.78% | 0.51% | 0.96% | 0.46% |
| ***hsa-miR-4530*** | 0.71% | 1.14% | 0.75% | 0.25% |
| ***hsa-miR-6782-5p*** | 0.59% | 0.77% | 0.90% | 0.16% |
| ***hsa-miR-6741-5p*** | 0.59% | 1.10% | 0.32% | 0.34% |
| ***hsa-miR-1307-5p*** | 0.43% | 0.77% | 0.64% | 0.02% |
| ***Other hsa-miR:*** | 22.71% | 28.70% | 25.41% | 17.39% |

Among the other sequenced miRs, the following were found in concentrations less than or equal to 0.02%, in all batches analyzed:
hsa-miR-8078; hsa-miR-7150; hsa-miR-6750-5p; hsa-miR-6727-5p; hsa-miR-5585-3p; hsa-miR-4459; hsa-miR-4449; hsa-miR-3687; hsa-miR-3197; hsa-miR-1915-3p; hsa-miR-1285-5p; hsa-miR-1275; hsa-miR-1273h-5p; hsa-miR-1273d; hsa-miR-1237-5p; hsa-miR-874-3p; hsa-miR-671-5p; hsa-miR-424-5p; hsa-miR-376c-3p; hsa-miR-339-3p; hsa-miR-222-3p; hsa-miR-221-3p; hsa-miR-199a-3p; hsa-miR-193a-3p; hsa-miR-181a-5p; hsa-miR-146a-5p; hsa-miR-145-5p; hsa-miR-143-3p; hsa-miR-130a-3p; hsa-miR-125b-5p; hsa-miR-125a-5p; hsa-miR-100-5p; hsa-miR-99a-5p; hsa-miR-92a-3p; hsa-miR-31-5p; hsa-miR-29b-3p; hsa-miR-29a-3p; hsa-miR-27b-3p; hsa-miR-27a-3p; hsa-miR-26a-5p; hsa-miR-24-3p; hsa-miR-23b-3p; hsa-miR-23a-3p; hsa-miR-22-3p; hsa-miR-19b-3p; hsa-miR-16-5p and hsa-let-7i-5p.

### Example 2: Application of the composition of the invention to the treatment of osteoarthritis.

To evaluate the effect of the composition of the invention on osteoarticular diseases (OA), the compositions obtained in example 1 were used interchangeably, in a model of murine osteoarthritis. In this model, the disease is induced by injecting collagenase into the joint, in this case knee, on days 0 and 2, and then on days 7 and 14 the respective treatment is applied, also by injection into the joint. Then, on day 40, euthanasia is performed, and the joint is analyzed.

In this case, 3 independent studies were conducted, in which a total of 65 animals were treated (Study 1: 17 animals, Study 2: 20 animals, Study 3: 28 animals).

Each group was subdivided into 3, a first control group, which received placebo (PBS, 5 µΛ), a second control group to which the lesion is induced, but the treatment is not applied, so they maintain the osteoarthritis disease (OA), and finally the group to which the lesion is induced and the invention treatment is applied. To induce the injury, 5 µL collagenase type VII, 1 U/5 µL, was injected into the joint at the beginning of the experiment and on day 2 of the experiment, for the injury-only (OA) groups and those subsequently treated. Subsequently, the animals of the third group received as treatment the composition of the invention adjusted in order to apply a quantity of 2×10⁸ exosomes, the treatment was applied 2 times, on days 7 and 14 from the beginning of the experiment, in a volume of 5µL
After the *in vivo* studies were completed, an analysis of the treated joint was performed, either with placebo, the lesion alone, or with treatment. To analyze the state of the joint, 2 techniques were used, the first, the study of images by means of a *microCT equipment,* which delivers the result of bone mineral density. A second option used was to perform a histological analysis of the treated knees, where an OA histology score was established, which corresponds to an indicator of the clinical signs of the murine model of osteoarthritis *(*Cosenza, S., Ruiz, M., Maumus, M., Jorgensen, C. & Noël, D. Pathogenic or Therapeutic Extracellular Vesicles in Rheumatic Diseases: Role of Mesenchymal Stem Cell-Derived Vesicles. Int. J. Mol. Sci. 18, (2017*).*

As indicated the inventors conducted 3 in vivo studies, the summary of the experiments is presented in Table 4, below. It should be noted that in In vivo N°1 the results were analyzed by histological analysis and *in vivo* 2 and 3 the results were evaluated using *microCT analysis.*

**Table 4 Summary of In vivo OA studies for the groups studied**

| OA No. | Treatment | N° of animals | MicroCT analysis | Histological analysis |
|---|---|---|---|---|
| 1 | UC-MSC Exosomes (2×10⁸) | 17 | | X |
| 3 | UC-MSC Exosomes (2×10⁸) | 20 | X | |
| 4 | UC-MSC Exosomes (2×10⁸) | 28 | X | |

### Results

With respect to *In vivo* study No. 1, the results are shown in Figure 1. It was observed that the treatment with the composition of the invention had an effect on the lesion, i.e. the *OA score* of the joint decreased with respect to the control without treatment (Figure 1A), showing a cartilage turnover and/or cartilage regeneration respectively, which would validate both that the *in vivo* model is working, and that the exosomes exhibit preventive and/or regenerative activity.

Understanding that this in vivo study corresponds to an isolated experiment, this data was reaffirmed with other studies, whose results were analyzed by *microCT.*

With respect to the *In vivo* study N°2 (*Figure* 2), according to the *microCT analysis, it* can be seen that the composition of the invention, or exosomes, produced by the inventors managed to prevent the damage generated by osteoarthritis, due to the fact that they have a lower number for the bone mineral density BMD (Figure 2A) than in the control with only the lesion, obtaining a value similar to that of the healthy joint treated with placebo. Specifically, the imaging analysis of bone mineral density (Figure 2B) showed that the induction model used generated a significant injury, despite this the exosomes would be able to generate a regenerative effect.

For the last *in vivo* study, Study 3, as can be seen in Figure 3, it can be observed that there is a significant effect between the composition of the invention or sEVs, obtained according to example 1 of UC-MSC cells, with respect to the lesion (OA). As with the previous *in vivos,* that the compositions according to the invention have preventive and/or regenerative effects for the disease of osteoarthritis is ratified. These results demonstrate that the application of the composition of the invention in an osteoarticular lesion such as osteoarthritis has a positive effect both on the degree of the lesion, as seen in histological studies and in the OA score, as well as on the bone mineral density of the joint.

### Example 3: Immunosuppression assay with the composition of the invention.

Mononuclear cells were isolated from peripheral blood, which were activated with PHA (20µg/mL) and stained with CTV (1:1000) to evaluate proliferation. Two experimental groups were considered: a) 10 µL of PBS control and b) treated with a single dose of the composition of the invention (sEV's) with a total particle quantity of 2×10⁸, dissolved in 10µL of vehicle. In both groups, the proliferation of helper T lymphocyte cells (abbreviated as Th; CD4+) and the percentage of regulatory T cells (abbreviated as Tregs; CD4+FOXP3+CD25+). Figure 4 shows the percentages of proliferation of Th lymphocyte cells on the third day of culture (Fig. 4a) and on the fifth day of culture (Fig. 4b), where it is observed that treatment with the composition of the invention produced less proliferation. Figure 5 shows the percentage of Treg cells, showing that treatment with the composition of the invention (sEV's) increased the percentage of this population on the third day of culture.

The Th cells correspond to a cell population with an important role in the adaptive immune response. These cells divide into Th1, Th2, and Th17; All of them secrete cytokines to stimulate the proliferation and differentiation of cells involved in the immune response. There is evidence that T cells are present in greater numbers in organs with OA than in healthy controls. Tregs, on the other hand, are a subpopulation of CD4+ T cells that maintain homeostasis and tolerance within the immune system, regulating or suppressing other CD4+ and CD8+ T cells, and perhaps B lymphocytes and dendritic cells as well. Tregs cells can produce molecules with immunosuppressive function such as TFG-β, IL-10, and adenosine.

### Example 4: Macrophage Polarization Assay with Invention Composition

Mononuclear cells were isolated from peripheral blood and then, using a negative selection kit, the monocytes present were obtained. These monocytes were cultured and differentiated into macrophages using M-CSF and GM-CSF factors. After six days of culture, two experimental groups were established: a) control and b) treated with a single dose of the sEV composition of the invention, with a total particle quantity of 1×10⁹.

24 hours after stimulation, flow cytometry was used to evaluate the percentages of pro-inflammatory macrophages (called M1; CD86+HLA-DR+) and anti-inflammatories (called M2; CD206+ HLA-DR+). Figure 6 shows the percentages of double-positive cells for the M1 (Figure 6a) and M2 (Figure 6b) markers.

The results show that sEV treatment decreases the population of M1 macrophages and increases the population of M2 macrophages.

## Claims

1. Composition enriched in extracellular vesicles (EV) of umbilical cord mesenchymal cells, **CHARACTERIZED in that** the composition consists of
- in a set of EVs containing the miRNAs: hsa-miR-6126, hsa-miR-149-3p and/or hsa-miR-6780b-5p, among the 10 most abundant miRNAs,
and additionally contain **at least 5** of the following miRNAs: hsa-miR-574-5p, hsa-miR-6131, hsa-miR-6741-5p, hsa-miR-21-5p, hsa-miR-1290, hsa-miR-4530, hsa-miR-2861, hsa-miR-6088, hsa-miR-1307-5p and/or hsa-miR-6782-5p among the 100 most abundant miRNAs,
and have the surface markers CD63, CD81, and CD9;
- and a medium or vehicle free of animal components and proteins, with an osmolality of between 250 and 310 mOsmol/L and a pH between 6.0 and 8.0.

2. Composition according to claim 1 **CHARACTERIZED in that** the vesicles contain at least 8 of the following miRNAs: hsa-miR-574-5p, hsa-miR-6131, hsa-miR-6741-5p, hsa-miR-21-5p, hsa-miR-1290, hsa-miR-4530, hsa-miR-2861, hsa-miR-6088, hsa-miR-1307-5p and/or hsa-miR-6782-5p among the 100 most abundant miRNAs.

3. Composition according to claim 1 **CHARACTERIZED in that** the vesicles additionally have the surface markers N-cadherin and/or CD90 and/or CD44.

4. Composition according to claim 1 **CHARACTERIZED in that** the vesicles are in a concentration of between 5×10⁷ to 1×10¹³ total particles per mL.

5. Composition according to claim 4 **CHARACTERIZED in that** the vesicles range in size from 30 to 300 nm.

6. Method of obtaining the composition of claim 1 **CHARACTERIZED in that** EVs between 30 and 300 nm are isolated from the supernatant of the culture of umbilical cord mesenchymal cells expanded in *vitro,* where such cells express at least 2 of the surface markers N-cadherin, CD44, RANKL, CD105, CD56 and/or CD90 and are resuspended in a medium or vehicle free of animal and protein components, with an osmolality between 250 and 310 mOsmol/L and a pH between 6.0 and 8.0.

7. Method according to claim 6 **CHARACTERIZED in that** the EVs are isolated from the supernatant by ultrafiltration, filtration, ultracentrifugation and/or centrifugation, washed, at least 2 times with a medium or vehicle free of animal components and proteins, with an osmolality between 250 and 310 mOsmol/L and a pH between 6.0 and 8.0, and finally they are resuspended in a medium or vehicle free of animal components and proteins, with an osmolality of between 250 and 310 mOsmol/L and a pH between 6.0 and 8.0.

8. Method according to claim 7 **CHARACTERIZED in that** umbilical cord mesenchymal cells express at least 3 of the surface markers N-cadherin, CD44, RANKL, CD105, CD56 and/or CD90.

9. Use of the composition of claim 1 **CHARACTERIZED in that** it is used to prepare a medicinal product useful in the treatment of osteoarticular and autoimmune diseases.

10. Use, according to claim 9 **CHARACTERIZED in that** the composition is useful in promoting cartilage regeneration.

11. Use, according to claim 9 **CHARACTERIZED in that** the composition is useful in preventing the degradation of cartilage.

12. Use, according to claim 9 **CHARACTERIZED in that** the composition is useful in decreasing bone mineral density in osteoarticular diseases.

13. Use, according to claim 9 **CHARACTERIZED in that** the composition is useful in decreasing the proliferation of CD4+ T cells.

14. Use, according to claim 9 **CHARACTERIZED in that** the composition is useful for increasing the proliferation of T regs cells.

15. Use, according to claim 9 **CHARACTERIZED in that** the composition is useful for increasing M2 macrophages, or CD206+ HLA-DR+ anti-inflammatory macrophages, and/or decreasing M1, or pro-inflammatory or CD86+HLA-DR+ macrophages.

16. Use, in accordance with claim 9 **CHARACTERIZED in that** the composition is useful for the treatment of osteoarthritis, arthritis, osteoarthritis,

17. Use, according to claim 9 **CHARACTERIZED in that** the composition is useful for the treatment of rheumatoid arthritis, systemic lupus erythematosus, inflammatory bowel disease, multiple sclerosis, type 1 mellitus diabetes, Guillain-Barre syndrome, chronic inflammatory demyelinating polyneuropathy, psoriasis, Graves' disease, Hashimoto's thyroiditis, myasthenia gravis, vasculitis, Sjögren's syndrome, pernicious disease anemia, celiac disease or graft-versus-host disease.
